# FASCICULE DE BREVET EUROPEEN

(11) **EP 3 027 036 B1**
(45) Date de publication et mention de la délivrance du brevet: **14.11.2018**
(21) Numéro de dépôt: 14748258.2
(22) Date de dépôt: 29.07.2014
(51) Int. Cl.: A23D 9/007, A23D 9/02, A61Q 19/00, A61K 35/00

(54) **PRODUIT A BASE D'HUILE D'ORIGINE VEGETALE HYPEROXYGENEE**
PRODUKT AUF DER BASIS VON HYPEROXYGENIERTEM ÖL PFLANZLICHEN URSPRUNGS
PRODUCT BASED ON HYPEROXYGENATED OIL OF PLANT ORIGIN

(30) Priorité: 01.08.2013 FR 1357623
(43) Date de publication de la demande: 08.06.2016
(73) Titulaire: Urgo Recherche Innovation et Développement, 21300 Chenove (FR)
(72) Inventeur: APERT, Laurent, F-21000 Dijon (FR)
(74) Mandataire: Cabinet Plasseraud
(86) Numéro de dépôt international: PCT/FR2014/051967
(87) Numéro de publication internationale: WO 2015/015116

(56) Documents cités:
- EP-A2- 0 225 831
- EP-A2- 1 077 061
- FR-A1- 2 591 112

## Description

La présente invention se rapporte à un nouveau produit comprenant au moins une huile d'origine végétale hyperoxygénée, la définition de cette huile en spectrophotométrie UV étant de 10 à 20 pour le facteur E270 et de 8 à 60 pour le facteur E232.

Bon nombre de documents de la littérature traitent des huiles ou corps gras hyperoxygénés ou peroxydés dans diverses utilisations thérapeutiques ou cosmétiques.

Parmi ces utilisations-ci, on peut retrouver notamment le traitement des escarres. Les escarres peuvent se caractériser comme des états de lésions cutanées d'origine ischémique liées à une compression des tissus mous entre un plan dur et des saillies osseuses. L'escarre est causée par une suppression de l'irrigation sanguine des tissus, suppression entrainant leur nécrose. Ce type de plaie se caractérise également par le fait de ne pas présenter de cicatrisation spontanée. La survenue de telles problématiques est particulièrement favorisée chez les personnes longuement alitées notamment chez les personnes en fin de vie, dans le coma ou encore paraplégiques. En plus de ces sujets présentant de fortes prédispositions à développer une telle pathologie, des facteurs aggravants peuvent venir s'ajouter, tels que la dénutrition, la déshydratation ou encore l'hyperthermie, et empêcher d'avantage encore le déroulement d'une cicatrisation efficiente.

De nombreuses solutions ont été apportées pour résoudre ce problème. La demande EP 0 293 535 décrit une composition particulière à base d'huile d'origine végétale hyperoxygénée et présentant un taux de peroxydation compris entre 50 et 120 milliéquivalents par kilogramme et une teneur en glycérides oxydés comprise entre 5 et 40, la définition de cette huile en spectrophotométrie UV étant de 1 à 6 pour le facteur E270 et de 8 à 60 pour le facteur E232 comme convenant tout particulièrement au traitement des escarres.

Il est à noter que ce produit décrit dans cette demande se trouve caractérisé notamment par une proportion importante de produits primaires d'oxydation émanant de l'huile ou des huiles d'origine végétale qu'il contient en contrepartie d'une plus faible proportion de produits secondaires d'oxydation. Cette proportion est gage d'instabilité de la composition et aboutirait à mettre en contact des substances hyper-réactives, voire cytotoxiques avec une peau elle aussi très réactive due à son statut pathologique.

Afin de déterminer la proportion de produits primaires d'oxydation et de produits secondaires d'oxydation, les biochimistes utilisent la méthode appelée méthode des diènes conjugués. Cette méthode est une méthode spectrométrique de dosage de ces deux types de composés, révélateurs du niveau d'oxydation de l'huile ou du mélange d'huiles présent dans la composition finale.

L'oxydation des acides gras polyinsaturés conduit à la formation de structures conjuguées qui absorbent de façon très spécifique dans l'ultra-violet, par exemple à 232 nm pour les structures diéniques et à 270 nm pour les structures éthyléniques.

La conclusion attribuée à ces mesures est que plus l'absorbance est élevée à 232 nm, plus la matière grasse est riche en produits primaires d'oxydation. A l'inverse, plus l'absorbance est élevée à 270 nm, plus la matière grasse est riche en produits secondaires d'oxydation.

Il est donc indispensable de posséder une huile d'origine végétale dont les produits d'oxydation primaires, notamment ceux des acides gras libres, sont remplacés par des produits d'oxydation secondaires, moins réactifs.

Il n'est pas connu à ce jour par la Demanderesse de produit comprenant une huile d'origine végétale hyperoxygénée présentant un taux avantageux en produits secondaires d'oxydation, notamment en raison du fait qu'il est particulièrement difficile de contrôler le stade d'oxydation de tels produits. En effet, à l'heure actuelle, il n'est connu aucune huile végétale hyperoxygénée définie par spectrophotométrie UV possédant pour le facteur E270 une valeur comprise hors des bornes 1 à 6 décrites jusqu'alors.

Il apparait donc nécessaire de disposer d'un produit simple de réalisation, prenant en compte le niveau d'oxydation de l'huile, notamment vis-à-vis de certains problèmes de cytotoxicité liés à cette dernière, et étant au moins aussi efficace que les produits existants à l'heure actuelle.

De façon tout à fait surprenante, il a été mis au point un nouveau produit à base d'huile d'origine végétale présentant un taux particulier en produits secondaires d'oxydation, composition qui se révèle capable de convenir particulièrement à la prévention et au traitement des escarres.

Ainsi la présente invention consiste en un nouveau produit comprenant au moins une huile d'origine végétale hyperoxygénée, la définition de cette huile en spectrophotométrie UV étant de 10 à 20 pour le facteur E 270 et de 8 à 60 pour le facteur E 232 (NF EN ISO 3656).

Cette huile végétale hyperoxygénée étant obtenue par saturation en oxygène et exposition intensive et contrôlée aux ultraviolets.

Selon un autre mode de réalisation de l'invention, celle-ci consiste en un procédé de fabrication dudit produit, caractérisé en ce que ledit procédé comprend les étapes de :
a- Introduction de l'huile native dans une cuve de préparation,
b- Oxygénation ménagée de ladite huile,
c- Conditionnement dudit produit obtenu à l'étape b).

Enfin, selon un dernier mode de réalisation de l'invention, celle-ci consiste en une utilisation de ce produit comprenant cette huile dans la prévention et le traitement des escarres.

### DESCRIPTION DETAILLEE DE l'INVENTION

La présente invention consiste en un nouveau produit comprenant au moins une huile d'origine végétale hyperoxygénée, la définition de cette huile en spectrophotométrie UV étant de 10 à 20 pour le facteur E 270 et de 8 à 60 pour le facteur E 232 (NF EN ISO 3656).

Plus précisément, la présente invention consiste en un nouveau produit comprenant au moins une huile d'origine végétale hyperoxygénée par saturation en oxygène et exposition intensive et contrôlée aux ultraviolets de façon à présenter un taux de peroxydation compris entre 30 et 300 exprimé en milliéquivalents par kilogramme et une teneur en glycérides oxydés comprise entre 5 et 40.

Ce produit possède des qualités très particulières liées à son niveau d'oxydation particulier. Il en résulte que l'huile ou le mélange d'huile inséré dans ce produit possède une très forte activité oxygénante, apaisante, tout en étant peu réactive au contact de la peau auprès de laquelle elle est apposée.

Par « huile d'origine végétale », on entend toute huile extraite d'un végétal génétiquement modifié ou naturel.

Par le terme « hyperoxygénation », on entend tout procédé permettant d'incorporer à l'huile végétale native des molécules d'oxygène par saturation en oxygène. L'huile végétale résultante peut être qualifiée d'hyperoxygénée. Elle peut présenter des molécules dites péroxydées.

Par le terme « péroxydation », on entend tout produit résultant du procédé d'hyperoxygénation de l'huile végétale native, incorporant des molécules d'oxygène et contenant un groupe fonctionnel de formule chimique générale R-O-O-R' (R et R' étant deux radicaux de nature chimique différente, O étant une molécule d'oxygène).

Selon un mode de réalisation préféré, l'huile ou le mélange d'huiles végétale(s) hyperoxygénée(s) présente un taux de péroxydation compris entre 50 et 150 exprimé en milliéquivalents par kilogramme.

Selon un autre mode de réalisation préféré de l'invention, l'huile ou le mélange d'huiles végétale(s) hyperoxygénée(s) possède une définition en spectrophotométrie UV de 12 à 18 pour le facteur E 270 et de 18 à 22 pour le facteur E 232.

L'huile ou le mélange d'huiles d'origine végétale est choisi parmi le liste non limitative constituée des huiles de maïs, des huiles d'amande douce, des huiles de carthame, des huiles de noisette, des huiles d'arachide, des huiles d'olive, des huiles de colza, des huiles de soja, des huiles d'Onagre, des huiles de tournesol, des huiles de pépin de raisin, ou encore des huiles de sésame.

De façon préférée, il est utilisé de l'huile de maïs.

Le produit objet de l'invention peut se présenter sous différentes formes, telle que par exemple sous la forme d'une huile, d'une crème, d'une émulsion, d'une pommade, d'une lotion, d'une solution, d'un spray, d'un gel, d'une lingette imprégnée, d'un pansement, d'une bande de contention imprégnée, d'une capsule ou encore d'un lait.

De façon préférée, le produit se présente sous la forme d'une huile ou d'une capsule.

Par « capsule » on entend tout article à coque dure ou molle capable d'être rompue par simple pression, comprenant le produit comprenant lui-même l'huile ou le mélange d'huile d'origine végétale hyperoxygénée(s) sous forme liquide.

Par « imprégnée », on entend toute lingette ou encore bande de contention mise au contact du produit liquide et absorbé ou adsorbé par ladite lingette ou bande de contention.

Selon un mode de réalisation particulier de l'invention, le produit comprenant l'huile ou le mélange d'huiles végétales comprend en outre un agent antioxydant et/ou un arôme.

Par agent antioxydant on entend tout composé choisi parmi la liste de composés comprenant de façon non limitative l'acétate de tocophérol (Vitamine E), ou encore l'acide ascorbique. De façon préférée, la vitamine E est le composé antioxydant préférentiellement utilisé dans le produit objet de la présente invention.

Par arôme, on entend tout composé permettant de contrebalancer le pouvoir olfactif des produits secondaires d'oxydation dérivés de l'huile ou du mélange d'huiles végétales hyperoxygénée(s). En effet, lesdits produits secondaires d'oxydation présentent une odeur très caractéristique, odeur que l'on assimile souvent à une odeur de rancissement. Parmi les arômes préférentiellement choisis dans le cadre de la présente invention, on retrouve les arômes d'origine naturelle et de façon encore plus avantageuse les arômes d'origine naturelle et végétale. A titre d'exemple, des arômes de banane, de mangue, de kiwi, de pamplemousse, d'anis, de badiane, d'orange peuvent être utilisés dans le cadre de la présente invention. De façon préférée, l'arôme d'anis et l'arôme de pamplemousse sont choisis pour être inclus dans le produit objet de l'invention. Lesdits arômes possèdent des propriétés olfactives atypiques et qui présentent l'avantage d'annihiler l'odeur de rancissement dû aux produits secondaires de l'oxydation de l'huile ou du mélange d'huiles compris dans le produit objet de l'invention.

Selon un autre mode de réalisation de l'invention, il peut être envisagé d'associer une huile animale à ce produit objet de la présente invention comprenant une huile végétale hyperoxygénée. A titre d'exemple, on peut donc citer les huiles de poisson, de foie de morue, de pied de boeuf, le saindoux ou encore le suif. Parmi ces huiles, il est préféré d'utiliser l'huile de foie de morue.

Selon un mode de réalisation préféré de la présente invention, le produit est obtenu par un procédé de fabrication comprenant les étapes de :
a- Introduction de l'huile native dans une cuve de préparation,
b- Oxygénation ménagée de ladite huile,
c- Conditionnement dudit produit obtenu à l'étape b).

L'étape d'oxygénation ménagée consiste à greffer sur les molécules de triglycérides constitutives de l'huile, des fonctions hydroperoxydes. Ces fonctions hydroperoxydes apportent indirectement de l'oxygène aux tissus cutanés lorsque le produit est étalé sur la peau fragile et/ou endommagée du patient. Cette étape est cruciale et spécifique au produit objet de l'invention. Elle présente notamment l'avantage d'être plus souple et plus sélective que les oxydations réalisées par ozonolyse. Elle est réalisée par simple barbotage d'air réalisée en fond de cuve, ce qui permet d'introduire l'oxygène dans le produit mais également d'homogénéiser ledit produit. Elle se déroule à une température de 80-85°C et sans inertage.

L'étape de conditionnement du produit obtenu à l'étape c), autrement dit du produit final, peut être réalisé par simple remplissage de flacons de volumes variables par exemple.

Dans ce cas-là, le volume de produit final introduit dans chaque flacon peut notamment être contrôlé par l'utilisation d'un système de dosage, comme par exemple une pompe volumétrique, un système pondéral, un système optique ou encore un système temps-pression.

Selon un mode de réalisation particulier, le produit objet de l'invention comprend en outre une étape intermédiaire de refroidissement de l'huile obtenue après l'étape b). Celle-ci se déroule de façon aisée par simple mise en contact du produit obtenu à l'air ambiant.

Selon un autre mode de réalisation de l'invention, le procédé objet de l'invention comprend une étape d'incorporation d'un arôme entre l'étape d'oxygénation ménagée de l'huile et l'étape de conditionnement ou entre l'étape de refroidissement de l'huile, si l'étape est présente, et l'étape de conditionnement. La quantité d'arôme ajoutée est faible, de sorte à ne retrouver que des traces de celui-ci dans le produit final et que celui-ci n'apporte pas ses propriétés spécifiques. De façon préférée, la quantité d'arôme retrouvée dans le produit final n'excède pas les 1% en poids par rapport au poids du produit final. De façon encore plus préférée, l'arôme ajouté est l'arôme d'anis. Cette étape est facultative dans le cas où l'huile obtenue après refroidissement subie une étape de raffinage (telle que décrite infra) complète.

Selon un autre mode de réalisation de l'invention, le procédé objet de l'invention comprend une étape de raffinage, étape préalable à celle du conditionnement du produit final ou à une éventuelle incorporation de l'arôme d'anis.

L'étape de raffinage a pour but de diminuer la teneur en produits d'oxydation trop réactifs du produit objet de l'invention. Elle peut être utile également pour désodoriser l'huile et ou la décolorer. Elle permet également de calibrer le produit et de rendre ainsi ses caractéristiques et ses propriétés davantage reproductibles. L'étape de raffinage comprend au moins deux étapes. La première étape comprend une étape de lavage alcalin (à partir d'un mélange d'eau et de soude ou de potasse), permettant de neutraliser l'acidité libre contenue dans le produit par régulation du pH. Elle se déroule sous agitation turbulente à une température de 80-85°C. En fin d'étape, l'huile est séparée de l'eau par exemple par décantation. La deuxième étape est basée sur un lavage à la vapeur sèche, par exemple à une température comprise entre 120 et 140°C. Cette étape permet à terme d'éliminer de la phase huileuse une partie significative des produits de dégradation contenus dans l'huile. Enfin une dernière étape, facultative, de traitement aux charbons actifs peut être envisagée. L'utilité de cette étape est de désodoriser l'huile ainsi que de la décolorer comme écrit *supra.*

Ainsi le procédé objet de la présente invention peut comprendre les étapes suivantes :
a-Introduction de l'huile native dans une cuve de préparation,
b-Oxygénation ménagée de ladite huile,
c-Refroidissement de l'huile obtenue à l'étape b),
d-Raffinage dudit produit obtenu à l'étape c)
e-Introduction d'un arôme (si le traitement au charbon actif n'a pas été réalisé à l'étape précédente)
f-Conditionnement dudit produit obtenu à l'étape d) ou e).

Selon un autre mode de réalisation de l'invention, le produit comprenant l'huile ou le mélange d'huiles hyperoxygénée(s) d'origine végétale possédant lesdites caractéristiques mentionnées ci-dessus est particulièrement utile pour la prévention et le traitement des escarres.

Selon un mode de réalisation particulier de la présente invention, le produit comprenant l'huile ou le mélange d'huiles hyperoxygénées (s) d'origine végétale possédant lesdites caractéristiques mentionnées ci-dessus peut se révéler particulièrement utile pour la prévention et le traitement des pieds secs et des cors calleux. Cette huile peut également être utile dans la prévention et le traitement des pieds secs du pied du diabétique.

### EXEMPLES

### EXEMPLE 1 : EVALUATION COMPARATIVE D'HUILES HYPEROXYGENEES SUR LE TRAITEMENT DES ESCARRES

Une étude a été menée sur 16 patients alités et possédant une peau présentant une escarre sacrée superficielle, c'est-à-dire présentant une destruction de l'épiderme, autrement qualifiée d'escarre de stade II.

Cette étude a été menée sur une durée de 15 semaines. 8 patients se sont vus appliquer une huile végétale hyperoxygénée classique, éventuellement associée à de l'huile de foie de morue, dont le facteur E 270 possède une valeur comprise entre 1 et 6 en spectrophotométrie UV et 8 autres patients se sont vus appliquer une autre huile végétale hyperoxygénée dont le facteur E 270 possède une valeur comprise entre 10 et 20 en spectrophotométrie UV.

Pour chaque patient, 3 applications quotidiennes de produit en question ont été réalisées sur la zone concernée à raison de massage réalisés par effleurement de la zone à traiter. Chaque patient a également été soumis à des changements de position fréquents.

A la fin des 15 semaines de traitement, les patients traités par le produit objet de la présente invention présentent des résultats on ne peut plus satisfaisants, les tissus de l'épiderme étant totalement réparés. En comparaison, les patients traités par l'huile végétale hyperoxygénée possédant une valeur comprise entre 1 et 6 en spectrophotométrie UV présentent un léger retard dans la mise en place du tissu épithélial, les cellules de l'épithélium commençant seulement à migrer des berges de la plaie pour proliférer.

### EXEMPLE 2 : EXEMPLE DE COMPOSITIONS

Produit objet de l'invention réalisé sous forme d'huile :

| | |
|---|---|
| Huile de maïs hyperoxygénée | 99% |
| Arôme d'anis | 1% |
| Vitamine E | traces |

Produit objet de l'invention réalisé sous forme d'émulsion :

| | |
|---|---|
| Mélange huile de maïs et huile d'olive hyperoxygéné | 99% |
| Arôme de pamplemousse | 1% |
| Vitamine E | traces |

### EXEMPLE 3 : PROCEDE DE FABRICATION D'UN PRODUIT A BASE D'HUILE D'ORIGINE VEGETALE ET NATURELLE HYPEROXYGENEE

Une quantité d'huile de 1600 litres est introduite dans une cuve en inox de volume 2000 litres. La température de l'huile est portée à une température avoisinant les 85°C, et ce sans mise sous inertage, c'est à dire à l'air libre. En complément, de l'effet d'oxydation de surface, une série de diffuseurs fait barboter de l'air sous forme de bulles au coeur de la cuve afin d'incorporer de l'oxygène d'une part et d'autre part d'homogénéiser l'ensemble de par les turbulences internes créées. Cet apport forcé et maîtrisé en air est couplé à une exposition au rayonnement UV (lampe spectre de 310 nm à 410 nm / / Rapport UVB/UVA = 1,4% // luminance comprise entre 400 et 900 cd/m²), d'une puissance de 100 W. L'énergie d'activation provient en grande partie de l'exposition à ce type de rayonnements UV.

La durée de traitement peut être variable, car le procédé tient compte de l'origine de l'huile de maïs, laquelle comme tout produit d'origine naturelle, présente une variabilité (terroir, année, procédé d'obtention).

La durée est au minimum de 30 heures mais peut être portée jusqu'à 50 heures. Une phase de refroidissement à température ambiante succède au traitement de photo-oxydation.

Un contrôle du taux d'hydropéroxyde (également appelé IP ou indice de peroxydation) peut être réalisé. Si l'indice de peroxydation est compris dans la fourchette allant de 50 meq, 150 meq, une quantité d'arôme, par exemple d'anis (inférieure 1 % en poids par rapport au poids du produit), est incorporée à l'huile, si l'indice de peroxydation est inférieur, l'étape de peroxydation se poursuit. Au cas où l'indice de peroxydation ne conviendrait pas, le lot serait détruit.

Une fois l'arôme d'anis incorporé, l'huile est introduite dans des flacons de volumes variables, c'est l'étape de conditionnement.

Le volume de produit final introduit dans chaque flacon peut notamment être contrôlé par l'utilisation d'un système de dosage, comme par exemple une pompe volumétrique, un système pondéral, un système optique ou encore un système temps-pression.

Il est à noter que le procédé objet de l'invention étant plus spécifique que les autres procédés retrouvés décrits dans la littérature, celui-ci génère moins d'espèces chimiques réactives, ainsi la stabilité du produit obtenu n'en est que meilleure.

L'une des principales caractéristiques de cette huile obtenue par ce procédé est d'une part son taux d'hydroperoxyde (taux de conversion) allant de 1,5 % pour un indice de peroxydation de 30 mEq et de 15 % pour un indice de peroxydation de 300 mEq, et préférentiellement de 2,5% pour un indice de peroxydation de 50 mEq et de 7,5 % pour un indice de peroxydation de 150 mEq.

De plus, cette huile peut être caractérisée par un taux de tri-acyl-glycérol hydroperoxydés, compris dans la fourchette de 1 % à 10%, de préférence dans la fourchette allant de 2,5 % à 7,5% et de manière encore plus préférée dans la fourchette allant de 4 à 6 %.

D'autre part, l'huile obtenue par ce procédé peut être caractérisée par son niveau de produits secondaires d'oxydation à l'aide d'une méthode de mesure de l'absorbance, niveau compris entre 10 et 20 pour le facteur E 270 et préférentiellement compris entre 12 et 18.

## Revendications

1. Produit comprenant au moins une huile d'origine végétale hyperoxygénée, la définition de cette huile en spectrophotométrie UV étant de 10 à 20 pour le facteur E 270 et de 8 à 60 pour le facteur E 232.

2. Produit selon la revendication 1 **caractérisé en ce que** l'huile d'origine végétale hyperoxygénée présente un taux de peroxydation compris entre 30 et 300 exprimé en milliéquivalents par kilogramme et une teneur en glycérides oxydés comprise entre 5 et 40.

3. Produit selon la revendication 2, **caractérisé en ce que** le taux de peroxydation de l'huile d'origine végétale hyperoxygénée est compris entre 50 et 150 exprimé en milliéquivalents par kilogramme.

4. Produit selon l'une quelconque des revendications précédentes **caractérisée en ce que** la définition de cette huile en spectrophotométrie UV est de 12 à 18 pour le facteur E 270 et de 18 à 22 pour le facteur E 232.

5. Produit selon l'une quelconque des revendications précédentes **caractérisée en ce que** l'huile d'origine végétale est choisie parmi le groupe constitué par les huiles de maïs, les huiles d'amande douce, les huiles de carthame, les huiles de noisette, les huiles d'arachide, les huiles d'olive, les huiles de colza, les huiles de soja, les huiles d'Onagre, les huiles de tournesol, les huiles de pépin de raisin, les huiles de sésame et de leurs mélanges.

6. Produit selon l'une quelconque des revendications précédentes **caractérisée en ce que** l'huile d'origine végétale est l'huile de maïs.

7. Produit selon l'une quelconque des revendications précédentes **caractérisée en ce que** ledit produit se présente sous la forme d'une huile, d'une crème, d'une émulsion, d'une pommade, d'une lotion, d'une solution, d'un spray, d'un gel, d'une lingette imprégnée, d'un pansement, d'une bande de contention imprégnée, d'une capsule ou encore d'un lait.

8. Produit selon l'une quelconque des revendications précédentes **caractérisée en ce que** ledit produit contient en outre de la vitamine E et/ou de l'anis.

9. Procédé de fabrication d'un produit selon la revendication 1, **caractérisé en ce que** ledit procédé comprend les étapes de :
a-Introduction de l'huile native dans une cuve de préparation,
b-Oxygénation ménagée de ladite huile,
c-Conditionnement dudit produit obtenu à l'étape b).

10. Procédé de fabrication selon la revendication 9 **caractérisé en ce que** ledit procédé comprend une étape intermédiaire de refroidissement du produit obtenu à l'étape b).

11. Procédé de fabrication selon la revendication 9 ou 10 **caractérisé en ce que** ledit procédé comprend une étape d'incorporation d'un arôme entre l'étape d'oxygénation ménagée de l'huile et l'étape de conditionnement ou entre l'étape de refroidissement de l'huile et l'étape de conditionnement.

12. Procédé de fabrication selon l'une quelconque des revendications 9 à 11, **caractérisé en ce que** ledit procédé comprend une étape de raffinage du produit obtenu.

13. Produit selon la revendication 1 à 8 pour son utilisation dans la prévention et le traitement des escarres.

14. Produit selon la revendication 1 à 8 pour son utilisation dans la prévention et le traitement des pieds secs et des cors calleux.

## Patentansprüche

1. Produkt, umfassend mindestens ein hyperoxygeniertes Pflanzenöl, wobei die Definition dieses Öls durch UV-Spektrophotometrie 10-20 für den Faktor E 270 und 8-60 für den Faktor E 232 ist.

2. Produkt nach Anspruch 1, **dadurch gekennzeichnet, dass** das hyperoxygenierte Pflanzenöl einen Peroxidgehalt zwischen 30 und 300, ausgedrückt in Milliäquivalenten pro Kilogramm, und einen Gehalt an oxidierten Glyceriden zwischen 5 und 40 aufweist.

3. Produkt nach Anspruch 2, **dadurch gekennzeichnet, dass** das hyperoxygenierte Pflanzenöl einen Peroxidgehalt zwischen 50 und 150 umfasst, ausgedrückt in Milliäquivalenten pro Kilogramm.

4. Produkt nach einem der vorangegangenen Ansprüche, **dadurch gekennzeichnet, dass** die Definition dieses Öls durch UV-Spektrophotometrie 12-18 für den Faktor E 270 und 18-22 für den Faktor E 232 ist.

5. Produkt nach einem der vorangegangenen Ansprüche, **dadurch gekennzeichnet, dass** das Pflanzenöl ausgewählt ist aus der Gruppe bestehend aus Maisölen, süßen Mandelölen, Distelölen, Haselnussölen, Erdnussölen, Olivenölen, Rapsölen, Sojaölen, Nachtkerzenölen, Sonnenblumenölen, Traubenkernölen, Sesamölen und Mischungen hiervon.

6. Produkt nach einem der vorangegangenen Ansprüche, **dadurch gekennzeichnet, dass** das Pflanzenöl ein Maisöl ist.

7. Produkt nach einem der vorangegangenen Ansprüche, **dadurch gekennzeichnet, dass** das Produkt in Form eines Öls, einer Creme, einer Emulsion, einer Salbe, einer Lotion, einer Lösung, eines Sprays, eines Gels, eines imprägnierten Wischtuches, eines Wundverbandes, eines imprägnierten Kompressionsverbandes, einer Kapsel oder in Form von Milch vorliegt.

8. Produkt nach einem der vorangegangenen Ansprüche, **dadurch gekennzeichnet, dass** das Produkt außerdem Vitamin E und/oder Anis enthält.

9. Verfahren zur Herstellung eines Produktes nach Anspruch 1, **dadurch gekennzeichnet, dass** das Verfahren die folgenden Schritte umfasst:
a - Einführen des nativen Öls in einen Vorbereitungsbehälter,
b - kontrollierte Oxidation des Öls,
c - Conditionieren des in Schritt b) erhaltenen Produkts.

10. Verfahren zur Herstellung nach Anspruch 9, **dadurch gekennzeichnet, dass** das Verfahren einen intermediären Kühlungsschritt des in Schritt b) erhaltenen Produkts umfasst.

11. Verfahren zur Herstellung nach Anspruch 9 oder 10, **dadurch gekennzeichnet, dass** das Verfahren einen Schritt des Einbringens eines Aromas zwischen dem Schritt der kontrollierten Oxidation des Öls und dem Schritt des Conditionierens oder zwischen dem Schritt der Kühlung des Öls und dem Schritt des Conditionierens umfasst.

12. Verfahren zur Herstellung nach einem der Ansprüche 9 bis 11, **dadurch gekennzeichnet, dass** das Verfahren einen Raffinationsschritt des erhaltenen Produkts umfasst.

13. Produkt nach einem der Ansprüche 1 bis 8 für die Verwendung zur Vorbeugung und Behandlung von Schorf.

14. Produkt nach einem der Ansprüche 1 bis 8 für die Verwendung zur Vorbeugung und Behandlung von trockenen Füßen und Corpus Callosum.

## Claims

1. Product comprising at least one hyperoxygenated oil of plant origin, the definition of this oil in UV spectrophotometry being from 10 to 20 for the E270 factor and 8 to 60 for the E232 factor.

2. Product according to claim 1, **characterised in that** the hyperoxygenated oil of plant origin has a degree of peroxidation of between 30 and 300 expressed in milliequivalents per kilogram and an oxidised glyceride content of between 5 and 40.

3. Product according to claim 2, **characterised in that** the degree of peroxidation of the hyperoxygenated oil of plant origin in between 50 and 150 expressed in milliequivalents per kilogram.

4. Product according to any one of the preceding claims, **characterised in that** the definition of this oil in UV spectrophotometry is 12 to 18 for the E270 factor and from 18 to 22 for the E232 Factor.

5. Product according to any one of the preceding claims, **characterised in that** the oil of plant origin is chosen from the group consisting of corn oils, sweet almond oils, safflower oils, hazelnut oils, peanut oils, olive oils, colza oils, soya oils, evening primrose oils, sunflower oils, grapeseed oils or sesame oils and mixtures thereof.

6. Product according to any one of the preceding claims, **characterised in that** the oil of plant origin is corn oil.

7. Product according to any one of the preceding claims, **characterised in that** said product is in the form of an oil, a cream, an emulsion, an ointment, a lotion, a solution, a spray, a gel, an impregnated wipe, a dressing, an impregnated restraint bandage, a capsule or a milk.

8. Product according to any one of the preceding claims, **characterised in that** said product also contains vitamin E and/or anise.

9. Method for manufacturing a product according to any one of the preceding claims, **characterised in that** said method comprises the steps of:
a - introducing the native oil into a preparation vessel,
b - controlled oxygenation of said oil,
c - packaging of said product obtained at step b).

10. Manufacturing method according to claim 9, **characterised in that** said method comprises an intermediate step of cooling the product obtained at step b) .

11. Manufacturing method according to claim 9 or 10, **characterised in that** said method comprises a step of incorporating an aroma between the step of controlled oxygenation of the oil and the packaging step or between the step of cooling the oil and the packaging step.

12. Manufacturing method according to any one of claims 9 to 11, **characterised in that** said method comprises a step of refining the product obtained.

13. Product according to claims 1 to 8 for use thereof in the prevention and treatment of pressure sores

14. Product according to claims 1 to 8 for use thereof in the prevention and treatment of dry feet and corns.
